# EUROPEAN PATENT SPECIFICATION

(11) **EP 1 097 937 B1**
(45) Date of publication and mention of the grant of the patent: **08.06.2005**
(21) Application number: 00128100.5
(22) Date of filing: 06.04.1990
(51) Int. Cl.: C07H 15/203, A61K 31/704, A61P 31/04, A61P 35/00

(54) **N-acyl derivatives of the LL-E33288 antitumor antibiotics**
N-Acylderivate der Antitumor-Antibiotika vom Typ LL-E33288
Dérivés n-acyles d'antibiotiques antitumoraux du type LL-E33288

(30) Priority: 14.04.1989 US 338928
(43) Date of publication of application: 09.05.2001
(62) Divisional of application: 90106601.9
(73) Proprietor: Wyeth Holdings Corporation, Madison, NJ 07940 (US)
(72) Inventor: Lee, May Dean-Ming, Monsey, New York 10951 (US)
(74) Representative: Walters, Philip Bernard William

(56) References cited:
- EP-A- 0 276 485
- EP-A- 0 289 030
- LEE M D ET AL: "CALICHEMICINS, A NOVEL FAMILY OF ANTITUMOR ANTIBIOTICS CHEMISTRY AND PARTIAL STRUCTURE OF CALICHEMICIN" JOURNAL OF THE AMERICAN CHEMICAL SOCIETY,US,AMERICAN CHEMICAL SOCIETY, WASHINGTON, DC, vol. 109, no. 11, 27 May 1987 (1987-05-27), pages 3464-3466, XP000113790 ISSN: 0002-7863
- M. D. LEE ET AL.: "Calichemicins, a novel family of antitumor antibiotics. 2. Chemistry and structure of calichemicin." J. AM. CHEM. SOC., vol. 109, 1987, pages 3466-3468, XP002162621
- M.M. SIEGEL ET AL.: "Nonionic surfactants used as exact mass internal standards for the 700-2100 Dalton mass range in fast atom bombardment mass spectrometry" ANAL. CHEM., vol. 62, 1990, pages 322-327, XP000990100

## Description

Lee et al, J. Am. Chem. Soc. 1987, 109, 3464 -3466, describes a novel family of antitumor antibiotics referred to as the calichemicins. There is a reference to calicheinicin γ₁^{I} and N-acetylcalichemicin γ₁^{I}, (which latter has been referred to also in the following teaching) but it is believed that the paper does not provide an enabling disclosure of the compounds of the present invention.

EP-A-0289030 (Bristol-Myers Squibb) discloses N-acetyl derivatives designated N-acetylesperamicins A₁. A₂ and A_{1b}. It is stated that these derivatives exhibit both antimicrobial and antitumor activity.

### SUMMARY OF THE INVENTION

The invention describes certain N-acyl derivatives of the α₂^{Br}, β₁^{Br}, γ₁^{Br}, α₂^{I}, β₁^{I}, γ₁^{I}, and δ₁^{I} components and of the N-acyl-dihydro derivatives of the α₂^{Br}, β₁^{Br}, γ₁^{Br}, α₂^{I}, β₁^{I}, γ₁^{I}, and δ₁^{I} components of the LL-E33288 antibiotic complex prepared by reacting the antibiotic with an unsubstituted or substituted acid anhydride acyl cation equivalent or acid chloride. These N-acyl derivatives are effective antibacterial and antitumor agents.

European Patent No 392376B (of which this application is a divisional) discloses
N-acetyl-LL-E33288δ₁^{I}
N-formyl-LL-E33288δ₁^{I}
N-acetyl-LL-E33288γ₁^{I} and
N-acetyl-dihydro-LL-E332887γ₁^{I}, and their preparation.

### DETAILED DESCRIPTION OF THE INVENTION

The family of antibacterial and antitumor agents, known collectively as the LL-E33288 complex, are described and claimed in European Patent Application Number EP-A-182152 and are used to prepare the N-acyl derivatives of this invention. The above application describes the LL-E33288 complex, the components thereof, namely,
LL-E33288α₁^{Br}, LL-E33288α₂^{Br}, LL-E33288α₃^{Br},
LL-E33288α₄^{Br}, LL-E33288β₁^{Br}, LL-E33288β₂^{Br},
LL-E33288 γ₁^{Br}, LL-E33288α₁^{I}, LL-E33288α₂^{I},
LL-E33288α₃^{I}, LL-E32288β₁^{I}, LL-E33288β₂^{I}, LL-E33288γ₁^{I}
and LL-E33288δ₁^{I}
and methods for their production by aerobic fermentation utilizing a new strain of Micromonospora echinospora ssp. calichensis or natural or derived mutants thereof. The proposed chemical structures of some of the above named components are disclosed in European Patent Application Number EP-A-182152 and are reproduced in Table I below:

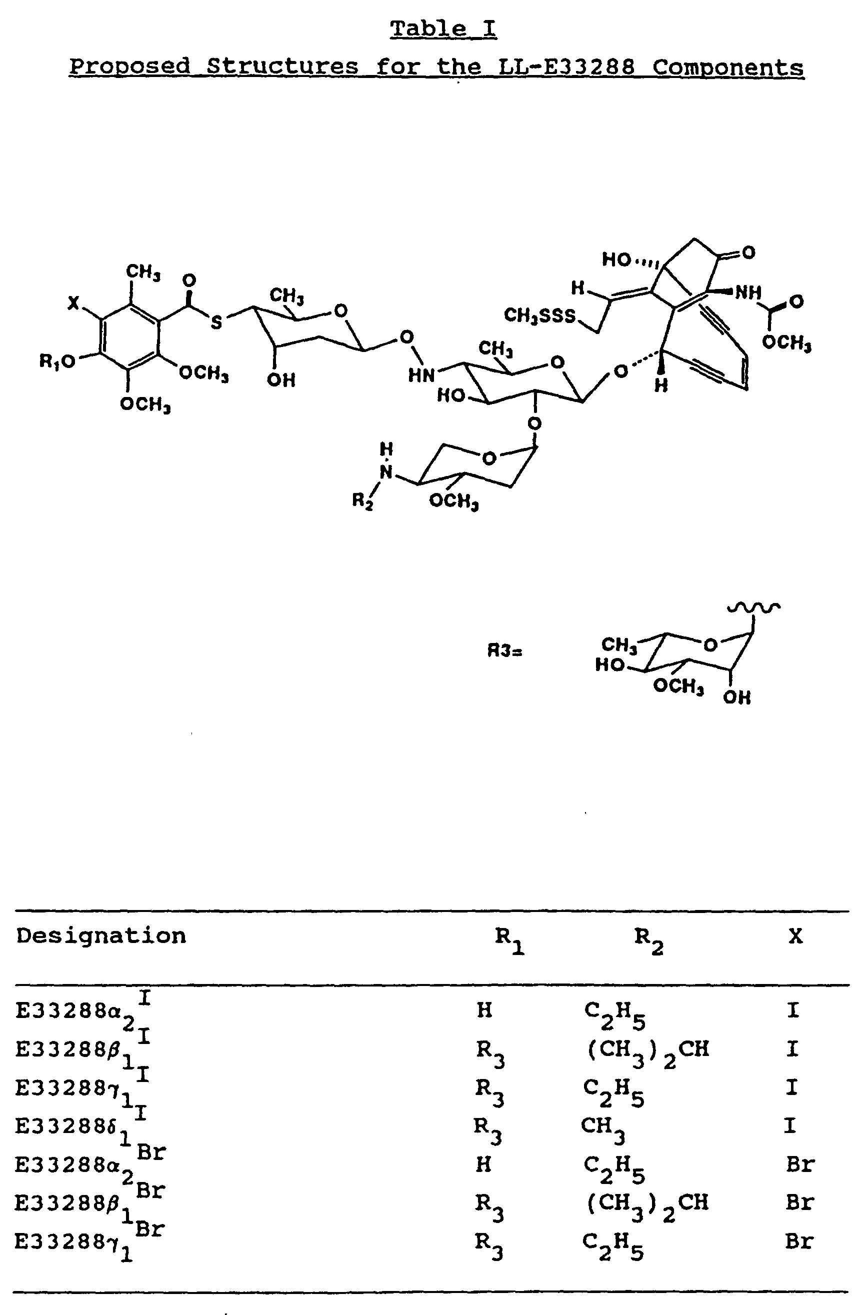

As can be seen from the structures disclosed in Table I, the α₂^{Br}, β₁^{Br}, γ₁^{Br}, α₂^{I}, β₁^{I}, γ₁^{I}, and δ₁^{I} components of the LL-E33288 antibiotic complex each contain a secondary amino group which is part of a substituted 4-aminopentose unit. It has now been discovered that the reaction of any of the above components with an unsubstituted or substituted, saturated or unsaturated alkyl or aryl acid anhydride, acid chloride or acyl cation equivalent results in the introduction of an acyl moiety on the secondary amino group as shown in Scheme I below. 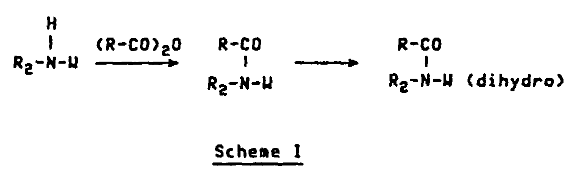 wherein W is the substituent attached to R₂NH- of the aminopentose in Table I, R is hydrogen or a branched or unbranched alkyl (C₁-C₁₀) or alkylene (C₁-C₁₀) group, an aryl or heteroaryl group, or an aryl-alkyl (C₁-C₅) or heteroaryl-alkyl (C₁-C₅) group, all optionally substituted by one or more hydroxy, amino, carboxy, halo, nitro, lower (C₁-C₃) alkoxy, or lower (C₁-C₅) thioalkoxy groups.

N-Acyl derivatives are also prepared from the dihydro derivatives of the LL-E33288 antibiotics, namely, dihydro-LL-E33288α₂^{Br}, dihydro-LL-E33288β₁^{Br}, dihydro-E33288γ₁^{Br}, dihydro-LL-E33288α₂^{I}, dihydro-LL-E33288β₁^{I}, dihydro-LL-E33288γ₁^{I}, and dihydro-LL-E33288δ₁^{I}, of parent application Serial No. 004,154.

As an example, reaction of LL-E33288γ₁^{I} with acetic anhydride in methanol produces N-acetyl-LL-E33288 γ₁^{I} while the reaction of LL-E33288 δ₁^{I} with the mixed anhydride of acetic acid and formic acid produces N-formyl-LL-E33288 δ₁^{I}, both potent new antitumor antibiotics. The reaction of dihydro-LL-E33288 E33288γ₁^{I} with acetic anhydride in methanol produces N-acetyl-dihydro-LL-E33288 γ₁^{I} . N-Acetyl-dihydro-LL-E33288 γ₁^{I} is also produced by the reaction of N-acetyl-LL-E33288 γ₁^{I} with sodium borohydride under the conditions described European Patent Application Number EP-A-276485.

The present invention provides N-acyl derivatives of LL-E33288 and dihydro LL-E33288 antibiotics of the formula 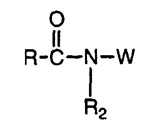 wherein W is 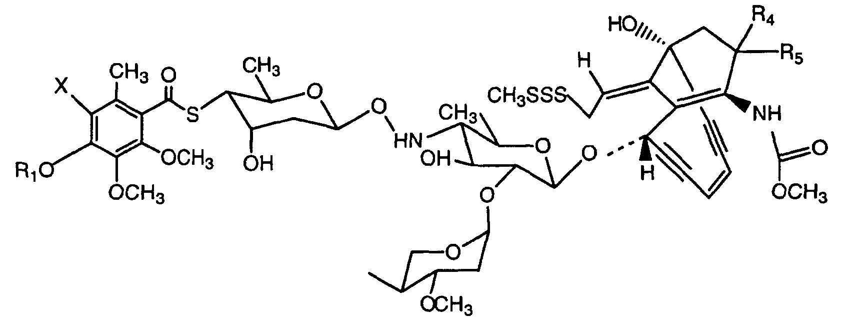 R is hydrogen or a branched or unbranched alkyl (C₁-C₁₀) or alkylene (C₁-C₁₀) group, an aryl or heteroaryl group, or an aryl-alkyl (C₁-C₅) or heteroaryl-alkyl (C₁-C₅) group, all optionally substituted by one or more hydroxy, amino, carboxy, halo, nitro, lower (C₁-C₃) alkoxy, or lower (C₁-C₅) thioalkoxy groups;
R₁ is H or 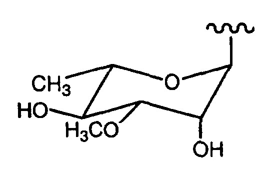 R₂ is CH₃, C₂H₅ or CH(CH₃)₂;
R₄ is OH when R₅ is H or R₄ and R₅ taken together are a carbonyl; and
X is an iodine or bromine atom, excluding:
N-acetyl-LL-E33288δ₁^{I};
N-formyl-LL-E33288δ₁^{I};
N-acetyl-LL-E33288γ₁^{I}
   and
N-acetyl-dihydro-LL-E33288γ₁^{I}.

The N-acyl derivatives of the LL-E33288 antitumor antibiotics are most conveniently characterized by high-performance liquid chromatography (HPLC) and by thin-layer chromatography (TLC).

The N-acyl derivatives of the LL-E33288 antitumor antibiotics are useful as antibacterial agents and are also active as antitumor agents as determined in the Biochemical Induction Assay (BIA), a bacterial assay system which specifically measures the ability of an agent to directly or indirectly initiate DNA damage.

This invention also provides a compound. of formula: 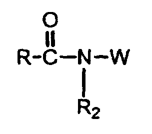 as defined above, for use in treating bacterial infections in warm-blooded animals, or for use in treating tumors in warm-blooded animals.

This invention also provides use of a compound of formula: 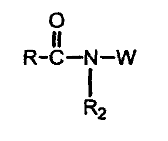 as defined above, in the preparation of a medicament for treating bacterial infections or for treating tumors in warm-blooded animals.

## Claims

1. A compound of the formula 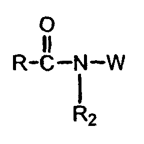 wherein W is 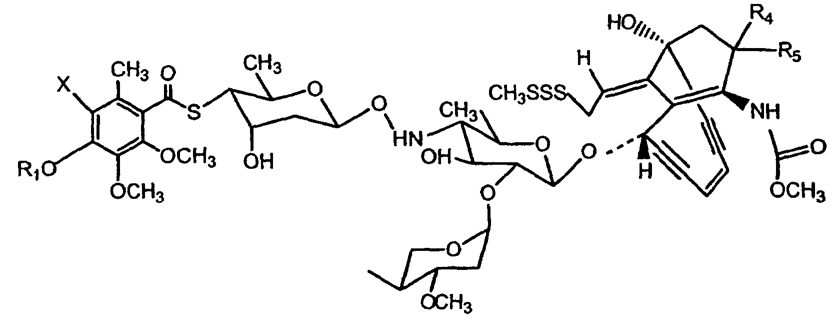 R is hydrogen or a branched or unbranched alkyl (C₁-C₁₀) or alkylene (C₁-C₁₀) group, an aryl or heteroaryl group, or an aryl-alkyl (C₁-C₅) or heteroaryl-alkyl (C₁-C₅) group, all optionally substituted by one or more hydroxy, amino, carboxy, halo, nitro, lower (C₁-C₃) alkoxy, or lower (C₁-C₅) thioalkoxy groups;
R₁ is H or 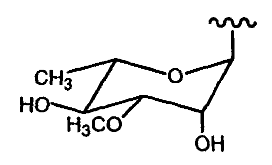 R₂ is CH₃, C₂H₅ or CH(CH₃)₂;
R₄ is OH when R₅ is H or R₄ and R₅ taken together are a carbonyl; and
X is an iodine or bromine atom, excluding:
N-acetyl-LL-E33288δ₁^{I};
N-formyl-LL-E33288δ₁^{I};
N-acetyl-LL-E33288γ₁^{I}
and
N-acetyl-dihydro-LL-E33288γ₁^{I}.

2. A compound according to Claim 1 for use in treating bacterial infections in warm-blooded animals.

3. A compound according to Claim 1 for use in treating tumors in warm-blooded animals.

4. Use of a compound according to Claim 1 in the preparation of a medicament for treating bacterial infections.

5. Use of a compound according to Claim 1 in the preparation of a medicament for treating tumors in warm-blooded animals.

## Patentansprüche

1. Verbindung der Formel 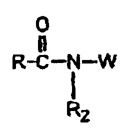 worin W für 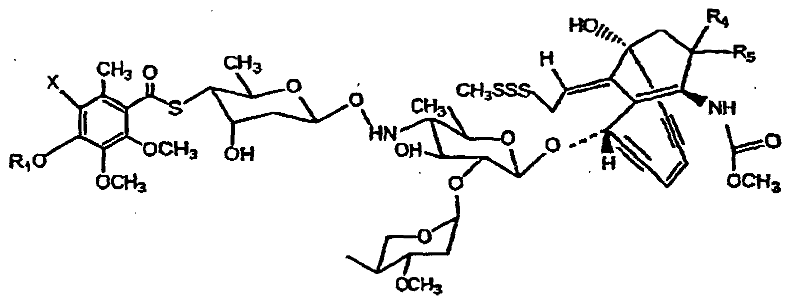 steht, R ein Wasserstoff oder eine verzweigte oder nicht verzweigte Alkyl(C₁-C₁₀)- oder Alkylen (C₁-C₁₀) gruppe, eine Aryl- oder Heteroarylgruppe oder eine Aryl-alkyl(C₁-C₅)- oder Heteroaryl-alkyl(C₁-C₅)gruppe darstellt, alle gegebenenfalls substituiert durch eine oder mehrere Hydroxy-, Amino-, Carboxy-, Halogen, Nitro-, nied.-(C₁-C₃)-Alkoxy- oder nied.-(C₁-C₅)-Thioalkoxygruppen;
R₁ für H oder 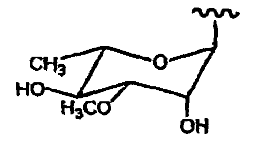 steht;
R₂ für CH₃, C₂H₅ oder CH(CH₃)₂ steht;
R₄ für OH steht, wenn R₅ für H steht, oder R₄ und R₅ zusammen genommen ein Carbonyl darstellen; und
X ein Iod- oder Bromatom darstellt, ausschließend:
N-Acetyl-LL-E33288δ₁^{I};
N-Formyl-LL-E33288δ₁^{I};
N-Acetyl-LL-E33288γ₁^{I}
und N-Acetyl-dihydro-LL-E33288γ₁^{I}.

2. Verbindung gemäß Anspruch 1 zur Verwendung beim Behandeln von bakteriellen Infektionen in warmblütigen Tieren.

3. Verbindung gemäß Anspruch 1 zur Verwendung beim Behandeln von Tumoren in warmblütigen Tieren.

4. Verwendung einer Verbindung gemäß Anspruch 1 bei der Herstellung eines Medikaments zum Behandeln von bakteriellen Infektionen.

5. Verwendung einer Verbindung gemäß Anspruch 1 bei der Herstellung eines Medikaments zum Behandeln von Tumoren in warmblütigen Tieren.

## Revendications

1. Composé de formule 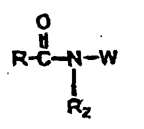 dans laquelle W est 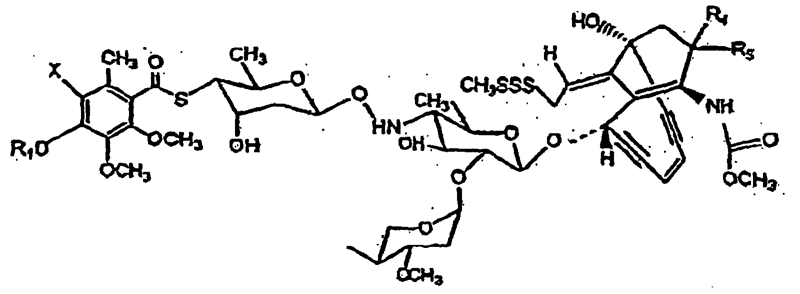 R est hydrogène ou un groupement alkyle en (C₁-C₁₀) ou alkylène en (C₁-C₁₀) ramifié ou non ramifié, un groupement aryle ou hétéroaryle, ou un groupement aryle-alkyle en (C₁-C₅) ou hétéroaryle-alkyle en (C₁-C₅), tous facultativement substitués par un ou plusieurs groupements hydroxy, amino, carboxy, halogène, nitro, alkoxy inférieur en (C₁-C₃), ou thioalkoxy inférieur en (C₁-C₅) ;
R est H ou 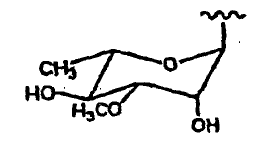 R₂ est CH₃, C₂H₅ ou CH(CH₃)₂;
R₄ est OH quand R₅ est H ou R₄ et R₅ pris ensemble sont un carbonyle, et
X est un atome d'iode ou de brome, à l'exclusion de :
N-acétyl-LL-E33288δ₁¹;
N-formyl-LL-E33288δ₁¹;
N-acétyl-LL-E33288γ₁¹;
et de N-acétyl-dihydro-LL-E33288γ₁¹.

2. Composé selon la revendication 1 pour une utilisation dans le traitement d'infections bactériennes chez des animaux à sang chaud.

3. Composé selon la revendication 1 pour une utilisation dans le traitement de tumeurs chez des animaux à sang chaud.

4. Utilisation d'un composé selon la revendication 1 dans la préparation d'un médicament pour le traitement d'infections bactériennes.

5. Utilisation d'un composé selon la revendication 1 dans la préparation d'un médicament pour le traitement de tumeurs chez des animaux à sang chaud.
